# EUROPEAN PATENT APPLICATION

(11) **EP 3 738 590 A1**
(43) Date of publication of application: **18.11.2020**
(21) Application number: 20177706.7
(22) Date of filing: 18.06.2014
(51) Int. Cl.: A61K 31/198, A23L 33/175, A61K 45/06, A61K 31/4172, A61P 21/00

(54) **DIETARY SUPPLEMENT COMPRISING AMINO ACIDS FOR THE TREATMENT OF SARCOPENIA**

(30) Priority: 31.07.2013 GB 201313635; 14.10.2013 GB 201318191
(62) Divisional of application: 14734541.7
(71) Applicant: Leeds Beckett University, Leeds LS1 3HB (GB)
(72) Inventor: KING, Roderick, Frederick, Gerardus, Joseph, Leeds, West Yorkshire LS21 2RG (GB); ISPOGLOU, Theocharis, Leeds, West Yorkshire LS8 2AL (GB); WHITE, Helen, Leeds, West Yorkshrie LS16 6HY (GB)
(74) Representative: Stuttard, Garry Philip

(57) **Abstract**

The present invention relates to a dietary supplement comprising a blend of essential amino acids (EEAs) which is rich in leucine and its use in combatting the degenerative loss of muscle characteristics which typify sarcopenia.

## Description

The present invention relates to a dietary supplement comprising a blend of essential amino acids (EEAs) which is rich in leucine and its use in combatting the degenerative loss of muscle characteristics which typify sarcopenia.

Sarcopenia is a complex, multi-factorial syndrome that is associated with significant clinical, social and economic consequences. With an ageing population, primary sarcopenia is becoming more common and estimated prevalence rates are 13% in 60 to 70 year-olds and up to 50% in people aged 80 years and over. Although there is no consensus for the definition of sarcopenia, the European Working Group on Sarcopenia in Older People (EWGSOP) have based diagnostic criteria on the presence of low muscle mass, together with either low muscle strength or low physical performance. Maintaining muscle mass and physical function is fundamental to promoting health, well-being and independence with age. It has particular relevance for the prevention of falls, fracture and disability. However the identification of effective treatments for age-related sarcopenia represents an ongoing challenge in research into ageing.

Some of the most promising and relevant interventions to date have been nutritionally-based. Adequate protein intake is considered to be essential. Recent reports suggest that the current recommended daily allowance (RDA) of 0.8 g/kg/day for the general and ageing population is too low. Instead prophylactic amounts of 1.0-1.6 g/kg/day have been proposed to meet the increased requirements of older people. It has also been suggested that the proportion of high quality protein in a meal should be increased rather than simply increasing general total protein intake. These reports together with the fact that protein intake can naturally decrease with age due to changes in taste, difficulties in chewing and swallowing and anorexia mean that there is considerable debate over the amount, quality and composition of protein sources acceptable for an elderly population. Research to determine the quantities, formulation, palatability and delivery of proteins and amino acids required for older people with existing sarcopenia or those deemed 'at risk' is considered to be a priority.

The amino acid L-Leucine (leucine) is associated with greater postprandial protein retention and thus better nitrogen balance than slow-release proteins. Leucine induces an up-regulation of mRNA translation, an increase in muscle protein synthesis and accretion and may reverse the blunt age-related response of muscle protein synthesis to increased protein intake. Longitudinal studies of nutritional intervention alone explored the effectiveness of essential amino acids (EAAs), leucine and EEAs enriched with leucine but presented conflicting findings and no conclusions on optimal supplementation protocols (see Dillon et al (2009) Amino acid supplementation increases lean body mass, basal muscle protein synthesis, and insulin-like growth factor-I expression in older women. J Clin Endocrinol Metab, 94, 1630-7; Verhoeven et al (2009) Long-term leucine supplementation does not increase muscle mass or strength in healthy elderly men. Am J Clin Nutr, 89, 1468-75; and Ispoglou et al (2011) Daily L-Leucine Supplementation in Novice Trainees During a 12-Week Weight Training Program. International Journal of Sports Physiology and Performance, 6, 38-50). The daily ingestion of 15g of a standard mixture of EAAs (20% leucine) over a period of 3 months improved lean body mass in older sedentary women (Dillon [*supra*]). In contrast a daily intervention of a high dose (7.5g) of free leucine had no effect on lean mass and showed no benefit over placebo with both groups increasing in lean tissue mass (LTM) by approximately 0.4 kg (Verhoeven [*supra*]). Neither intervention resulted in an increase in strength. Studies elsewhere however have demonstrated the potential of L-leucine as a pharmaconutritional prophylaxis against age-related sarcopenia but the amount and formulation of the most effective mixtures remains unclear (see Rieu et al (2006) Leucine supplementation improves muscle protein synthesis in elderly men independently of hyperaminoacidaemia. J Physiol, 575, 305-15; Rieu et al (2003) Leucine-supplemented meal feeding for ten days beneficially affects postprandial muscle protein synthesis in old rats. J Nutr, 133, 1198-205; Dardevet et al (2000) Stimulation of in vitro rat muscle protein synthesis by leucine decreases with age. J Nutr, 130, 2630-5; Guillet et al (2004) Mitochondrial and sarcoplasmic proteins, but not myosin heavy chain, are sensitive to leucine supplementation in old rat skeletal muscle. Exp Gerontol, 39, 745-51; and D'Antona et al (2010) Branched-chain amino acid supplementation promotes survival and supports cardiac and skeletal muscle mitochondrial biogenesis in middle-aged mice. Cell Metab, 12, 362-72).

The present invention is based on the recognition that a standard essential amino acid supplement enriched with high levels of leucine but not at the expense of other branched chain amino acids (BCAAs) has an improved efficacy on muscle mass and physical performance in the elderly.

Thus viewed from a first aspect the present invention provides a dietary supplement comprising (eg consisting essentially of or consisting of) a dry blend of essential amino acids being:
leucine in an amount in excess of 20wt% of the dry blend;
isoleucine and valine in a combined amount of 23wt% or more of the dry blend; and additional essential amino acids.

The dietary supplement of the invention administered orally to a subject is responsible for significant and advantageous gains in functional performance and/or lean tissue mass.

Preferably the amount of leucine is 25wt% or more of the dry blend, particularly preferably 30wt% or more of the dry blend, more preferably 35wt% or more of the dry blend, yet more preferably 40wt% or more of the dry blend.

Preferably the ratio of the amount of leucine to the combined amount of isoleucine and valine is greater than 0.870. Particularly preferably the ratio of the amount of leucine to the combined amount of isoleucine and valine is 1.739 or more.

Preferably the amount of isoleucine is in the range 9 to 13wt% of the dry blend.

Preferably the amount of isoleucine is 1 1wt% or more of the dry blend.

Preferably the amount of valine is in the range 10 to 14wt% of the dry blend.

Preferably the amount of valine is 12wt% or more of the dry blend.

Preferably the additional essential amino acids are (or include) histidine, lysine, methionine, phenylalanine and threonine.

Preferably the amount of histidine is 10wt% or less of the dry blend.

Preferably the amount of histidine is in the range 5 to 10wt% of the dry blend.

Preferably the amount of lysine is 15wt% or less of the dry blend.

Preferably the amount of lysine is in the range 12 to 15wt% of the dry blend.

Preferably the amount of methionine is 3wt% or less of the dry blend.

Preferably the amount of methionine is in the range 2 to 3wt% of the dry blend.

Preferably the amount of phenylalanine is 15wt% or less of the dry blend.

Preferably the amount of phenylalanine is in the range 7 to 15wt% of the dry blend.

Preferably the amount of threonine is 14wt% or less of the dry blend.

Preferably the amount of threonine is in the range 11 to 14wt% of the dry blend.

The dietary supplement may be formulated in a solid (*eg* a powder such as a rehydratable dehydrated powder) or a liquid (*eg* a suspension or emulsion) form which is physiologically tolerable.

The dietary supplement may be formulated in an orally administrable (*eg* ingestible) form such as a tablet, capsule or foodstuff (*eg* food or drink). The foodstuff may be chewable. The foodstuff may be a bar, biscuit, cookie, cake, sugar confectionery, gum, cereal or gel. For these purposes, the dietary supplement may further comprise one or more nutrients (*eg* vitamins), agents for promoting palatability (*eg* flavourings), colourants, adjuvants, excipients, gelating agents, binders, fillers, diluents, minerals, preservatives, sweeteners, emulsifiers, pharmaceuticals, viscosity modifiers, stabilisers, carriers or surfactants.

The amount of the dry blend of essential amino acids in the dietary supplement may be 40% or more.

Viewed from a further aspect the present invention provides dietary supplement as hereinbefore defined for use by oral administration to a subject in combatting (*eg* treating or preventing) degenerative loss of a muscle characteristic in the subject.

The muscle characteristic may be one or more of muscle mass, muscle quality or muscle strength. The degenerative loss of a muscle characteristic may be attributable to sarcopenia.

Preferably the muscle characteristic is muscle mass.

The dietary supplement for use is preferably by oral administration to the subject of doses of the dry blend in the range 0.16 to 0.30 g/kg (body weight) per day, particularly preferably in the range 0.18 to 0.24 g/kg (body weight) per day (eg about 0.21 g/kg (body weight) per day).

The dietary supplement for use is preferably by oral administration to the subject twice daily.

The dietary supplement for use is preferably by oral administration to the subject with food (*eg* with a meal).

The subject may be an elderly subject. For example, the subject may be aged 65 or more (*eg* in the range 65 to 75 years old).

Typically the subject is well nourished. Preferably the subject is receiving dietary protein in the range 0.6 to 1.3 g/kg (body weight) per day, particularly preferably 0.7 to 1.3 g/kg (body weight) per day, more preferably 1.0 to 1.3 g/kg (body weight) per day, yet more preferably 1.0 to 1.1 g/kg (body weight) per day.

The dietary supplement for use is preferably by oral administration to the subject over a period during which the subject is participating in exercise intervention.

The dietary supplement for use is preferably by oral administration to the subject over a period of 12 weeks or more.

Viewed from a yet further aspect the present invention may provide dietary supplement as hereinbefore defined for use by oral administration to a subject in improving the functional performance of the subject.

Viewed from a yet still further aspect the present invention provides dietary supplement as hereinbefore defined for use by oral administration to a subject in increasing bone mineral density (BMD) in the subject.

Viewed from an even yet further aspect the present invention may provide a method of treatment of a subject to combat (*eg* treat or prevent) degenerative loss of a muscle characteristic in the subject comprising: orally administering to the subject a dietary supplement as hereinbefore defined.

Viewed from an even still yet further aspect the present invention provides a method of treatment of a subject to improve the functional performance of the subject comprising: orally administering to the subject a dietary supplement as hereinbefore defined.

Viewed from a still further aspect the present invention provides a method of treatment of a subject to increase bone mineral density (BMD) in the subject comprising: orally administering to the subject a dietary supplement as hereinbefore defined.

The present invention will now be described in a non-limitative sense with reference to an Example.

### EXAMPLE

### Study Design

The study was a double-blinded randomised controlled pilot trial of the effects of mixtures of essential amino acids in men and women in their early retirement years. The study was approved by the University Research Ethics Committee and conducted in accordance with the Declaration of Helsinki. Signed informed consent was provided by all subjects prior to testing and intervention.

### Subjects/Population

Thirty six men and women aged 65 to 75 years volunteered to take part in the study and completed a standardised health screening questionnaire. Those with lactose intolerance, vascular disease, hypertension, diabetes, cardiac abnormalities and any other metabolic disease or injury were excluded from the study (n=3). Six subjects withdrew from the study and data from two subjects were not included in the final analysis due to non-compliance with the experimental protocol. Participants had not used nutritional supplements for the previous 3 months and only mild hypertensive medication was permitted. Twenty five men and women completed all tests (see Table 1 for age and anthropometric characteristics). None of the participants smoked. All of the participants were living independently and generally of good health.

### Protocol

Subjects received one of the following three supplements over a 3 month period:
A) Standard EEA mixture (0.21 g/kg BW) containing approximately 20% of L-Leucine
B) Enriched EEA mixture (0.21 g/kg BW) containing approximately 40% of L-Leucine
C) Isocaloric placebo containing lactose (0.21 g/kg BW).

In accordance with EWGSOP recommendations, the primary outcomes were total lean tissue mass and physical performance. All measurements were taken at baseline and immediately post intervention at 3 months. Before each testing session subjects had to complete a pre-exercise screening questionnaire and have their blood pressure (mmHg), resting heart rate (bpm) and oxygen saturation levels (%) measured. Following screening and before supplementation, all participants underwent functional performance testing that included the strength and endurance components of the Senior Fitness Test and The Handgrip Strength Test. Body composition testing included a dual energy X-ray absorptiometry (DXA) scan (Lunar iDXA, GE Healthcare, Madison, WI). Venous blood samples were drawn from a subgroup of subjects within each group to determine whether addition of extra leucine leads to a decrease in the level of other plasma amino acids and whether participants complied with the ingestion protocol.

### Supplements and Diet

### Supplements

To prevent satiation and gastric distension, the subjects ingested daily supplements in clear gelatine capsules (Size 00, CapsulCN) for a period of 3 months with their breakfast and dinner. A decision was made to use a dose of 0.21 g/kg BW instead of an absolute amount in an attempt to prevent excess or inadequate intakes for male and female participants. Capsules were indistinguishable by appearance, taste or scent and capsule dose ranged from 16 to 30 per day. Compliance was measured by recording the number of unused capsules at the end of the supplementation period. If subjects missed a bolus of the daily supplement, they did not have to take more capsules during the day to compensate. At baseline, subjects ingested the first dose of capsules to facilitate safe capsule ingestion and enable them to make an informed decision regarding their continuation with the study.

The EEAs and lactose (Arndale Ingredients, UK) were packed into 25 kg drums (Direct Food Ingredients Ltd, UK) to ensure homogeneity and then placed in capsules using a manual capsule machine (CN-300, CapsulCN) in the nutrition laboratory. The composition of the standard mixture of EAAs mirrored the amino acid composition of whey protein and provided 3g of leucine per 15g:
Histidine (10%)
Isoleucine (11%)
Leucine (20%)
Lysine (15%)
Methionine (3%)
Phenylalanine (15%)
Threonine (14%)
Valine (12%).

The mixture of EAAs enriched with leucine (40%) provided 6g of leucine per 15g:
Histidine (5%)
Isoleucine (11%)
Leucine (40%)
Lysine (12%)
Methionine (2%),
Phenylalanine (7%)
Threonine (11%)
Valine (12%).

The enriched mixture had the same percentage of non-leucine BCAAs as the standard mixture. The amount of leucine ingested by the participants was well below the upper tolerable and safe limit in humans which is set at 0.53 g/kg/day. All of the supplements were matched in terms of calorific content.

### Diet

During the first week of supplementation, participants were asked to record in detail by weight or by household measure all food and drinks consumed over a 3-day period and to retain all packaging and food labels where this was appropriate. Guidance on recording of household measures was provided. Where uncertainty existed regarding food measures, participants were contacted to check the accuracy of information. The same process was undertaken at follow-up on completion of the 3 month supplementation period. All food diaries were coded and dietary analysis was undertaken using NETWISP 3 dietary analysis software. Where food items were not available, recipes were inputted into the programme using ingredients provided by participants or from retained manufacturer food labels. Macronutrient intakes of protein, fat, carbohydrate and alcohol were obtained, each expressed as a percentage of macronutrient intake. Amounts of protein (g), iron (mg), calcium(mg) and vitamin D (ug) were also collected and expressed as a percentage of Reference Nutrient Intake (1991).

### Assessment of Body Composition

For anthropometric and body composition measurements, participants were measured wearing light weight clothing with jewellery removed. Height was determined with a stadiometer (SECA, Birmingham, UK) to the nearest millimetre and body weight was recorded by calibrated electronic scales (SECA, Birmingham, UK) to the nearest 0.1 kg. Body mass index (BMI) was calculated as body mass in kilograms/ height in metres squared. Body composition was assessed using DXA of the total body which provided a three component model of lean, fat and bone mass. Precision for measurements is 0.5%, 0.8% and 0.6% for lean, fat and bone mass respectively. The calibration of the machine was checked and passed on a daily basis using the GE Lunar calibration phantom. There was no significant drift in calibration throughout the duration of the trial.

### Functional Performance Testing and Physical Activity Levels

Subjects undertook four functional performance tests at baseline and at the end of the intervention period. These standardised tests had three components from the Senior Fitness Test [30-second Chair Stand Test (30-CST), the 30-second Arm Curl Test (30-ACT) and the 6-minute Walk Test (6-WT)] and the Handgrip Strength Test. The Senior Fitness Test has been recommended by the American College of Sports Medicine as appropriate to use with older adults and has been shown to correlate well with other muscular fitness tests such as one repetition maximum strength (1RM). The Handgrip Strength Test has been used previously with the older population in the Allied Dunbar National Fitness Survey and therefore allowed comparisons with the UK norms.

Subjects were tested in the following order:
1. 30-ACT
2. 30-CST
3. HST
4. 6-WT

The technique adopted during testing has been described previously (American College of Sports, M., Roitman, J. L. & Herridge, M. (2001) ACSM's resource manual for Guidelines for exercise testing and prescription, Philadelphia, Lippincott Williams & Wilkins; and Rikli, R. E. & Jones, C. J. (2013) Senior fitness test manual, Champaign, IL, Human Kinetics). Subjects had the opportunity to familiarise themselves with the equipment and perform sub maximum efforts before maximum testing at 30-ACT, 30-CST and HST. During the HST participants completed two maximum efforts with two minutes recovery between attempts. Ratings of perceived exertion were recorded at the end of each exercise and attempt (see Borg (1982) Psychophysical bases of perceived exertion/Les bases psychophysiques de la perception de l' effort. Medicine & Science in Sports & Exercise, 14, 377-381). A 5 lbs (2.3 kg) and an 8 lbs (3.6 kg) dumbbell (Neoprene) were used to measure number of repetitions performed by women and men respectively during the 30-ACT. The total number of complete chair stands during the 30-CST was recorded using a 44 cm height straight-back chair without arm rests that was placed against a wall for stability. A handheld dynamometer was employed to measure isometric strength (kg) of the dominant arm. During the 6-WT subjects were asked to walk as much of a set distance (50m, 20x5m rectangle with cones every 2 m) in 6 minutes.

A 7-day physical activity recall (7-d PAR - Hayden-Wade et al (2003) Validation of the telephone and in-person interview versions of the 7-day PAR. Med Sci Sports Exerc, 35, 801-9) which is considered to be a reliable self-reported method to assess physical levels was employed to identify differences in physical activity levels between groups at the start and end of the intervention.

### Blood Sampling and Analysis

Two mL of blood was drawn from antecubital vein at baseline and at the end of the experimental period from a sub-group of subjects (n=5, n=4 and n=6 from groups A, B, and C respectively volunteered to give blood samples) to measure plasma concentrations of the BCAAs (isoleucine, leucine and valine) and of the non-essential amino acids (NEAAs) alanine, glycine and proline. Subjects remained seated in a reclined position for 5 minutes before the sample was drawn from one of the brachial, medial cubital or radial veins. The samples were drawn into a vacutainer before being centrifuged for 10 minutes to separate plasma from blood cells. Two aliquots of plasma were stored at -80 °C before being analysed for plasma amino acids at the Scottish Universities Environmental Research Centre (East Kilbride). Each plasma sample was thawed, an internal standard added and then subjected to ultrafiltration to remove proteins. The ultrafiltrate was acidified and the sample subjected to cation exchange clean up, with elution in ammonium hydroxide. Samples were then dried down and derivatised for GCMS analysis.

These measurements were carried out in an attempt to monitor compliance to the supplementation protocol and to investigate the impact of particularly enhanced dietary L-Leucine intake on the plasma concentration of other amino acids. Due to non-compliance and drop-outs the final sample size of the subjects who provided samples at baseline and at the end of the intervention was n=3, n=4 and n=6 for groups A, B, and C respectively.

### Statistics

For all statistical analyses the SPSS statistical software was used (PASW 18). Data are presented as means (± standard deviations). Data was assessed for normality using the Shapiro-Wilk Test and data violating homogeneity assumptions was assessed via a Kruskal-Wallis test. One way ANOVA was used to test for differences at baseline and to compare mean percentage differences. Paired t-tests were used to compute within comparisons and for the non-parametric data (including RPE), the Wilcoxon signed rank test was used. Significant interactions were explored using the Holm-Bonferonni post-hoc adjustment where appropriate. Statistical comparisons for body composition, functional performance variables and RPE were performed using Cohen's Effect size (ES) with threshold values for small (0.2), medium (0.6), large (1.2), very large (2.0) and extremely large (4.0) effects. Statistical significance was also inferred as p<0.05.

### RESULTS

### Demographic Data

The age and anthropometric characteristics of the subjects are shown in Table 1 below. No significant differences were observed between groups in any of the characteristics at baseline. Weight was noted to be the lowest for Group C at baseline. However this was not reflected in BMI due to the fact that Group C had a lower stature. No significant differences were observed from baseline to 12 weeks for weight or BMI for Group A, B or C and no differences were observed between groups.

### Body Composition

Body composition variables are reported in Table 2 below. The only group that showed significant gains in LTM compared to baseline was group B [t (7) = -2.695, p=0.031] and this was accompanied by a small effect size (see Table 2). Changes in bone mineral density (BMD) may have not been significant between groups however percentage mean gains in BMD for groups A and B were associated with a large effect size. Overall no significant differences or an interaction effect were observed between the groups following 3 months of supplementation.

### Functional Performance (FP), Physical Activity (PA) and Ratings of Perceived Exertion (RPE)

FP and average RPE ratings for all exercises are given in Table 3 below. Baseline differences between groups A and C in the 30-second Chair Stand Test (30-CST) [F(2,22)=6.3, p=0.007] remained significant at the end of the intervention [F(2,22)=4.1, p=0.03]. There were no significant differences between groups in the mean percentage changes in scores for the 30-second Arm Curl Test (30-ACT) [F(2,22)=1.776, p=0.193], the 30-CST [F(2,22)=0.781, p=0.470], the relative Handgrip Strength Test (HST) [F(2,22)=1.271, p=0.300] and the 6-minute Walk Test (6-WT) [F(2,22)=2.225, p=0.132]. In contrast, the mean percentage reductions in RPE were significantly different between groups [F(2,22)=4.683, p=0.020]. Group C had the greatest reduction in RPE by the end of the intervention which was also significantly different from the reduction for the group A (p=0.023).

Compared to baseline only groups A and B demonstrated significant improvements in FP variables (see Table 3). In Group A, significant changes were observed in the 30-CST [t(7)=-3.211, p=0.015)] and 6-WT [t(7)=-2.859, p=0.024)]. In Group B, significant gains were observed in the 6-WT [t(7)=-2.659, p=0.032] and almost approached significance levels in the 3-CST [t(7)=-2.173, p=0.066]. The mean improvements in FP tests in the experimental groups were also associated with medium to large effects sizes in all exercises (see Table 3). RPE was reduced in Group C by the end of the intervention (Z=-2.668, p=0.008).

Overall PA at baseline was similar across all groups [F(2,22)=0.215, p=0.808] with the total duration spent in working and non-working related activities being 7.4 h (±7.4), 8.97 h (±6.85) and 7.14 (±3.71) for the A, B and C groups respectively. By the end of the intervention, groups A, B, and C increased their total PA levels on average by 104.7% (±344.3), 30.6% (±107.2) and 114.3% (±128.4) respectively. From a total of 8 subjects in Group A, 4 increased, 1 maintained and 3 decreased their PA levels. In Group B, 3 increased, 1 maintained and 4 decreased their PA levels. In the placebo Group C, 7 increased and 2 reduced their PA levels. Despite the majority of subjects in the placebo group increasing their PA levels, mean percentage changes were not significantly different between groups [F(2,22)=1.919, p=0.170]. However paired t-tests showed that Group C significantly increased total PA levels compared to baseline [(t(8)=-2.361, p=0.046)].

### Nutrition

Percentage capsule intake was 75.0% (±13.2), 75.8% (±10.4) and 85.8% (±15.0) for the groups A, B and C respectively indicating lower adherence levels for the experimental groups compared to placebo. Nevertheless significant differences were not observed between groups [F(2,22)=1.818, p=0.186]. Energy (kcal) intakes as a function of recommended daily estimated average requirements were suboptimal for all groups, although no significant differences existed between groups (Table 4 below). In contrast, protein intake for all groups exceeded the Reference Nutrient Intake (see Table 4). Relative protein intakes at baseline and at the end of the intervention ranged between 1.0-1.1 g/kg/day with no significant differences between groups. Percentage fat intake as a function of overall dietary energy remained within UK recommendation (<35% fat) for all groups indicating low fat intakes overall (see Table 4). The only difference between groups was in carbohydrate intake as a percentage of overall dietary energy intake. Group C (placebo) had greater % carbohydrate intake at baseline (46.9%) than both experimental groups [Group A, 36.9%; Group B, 41.7%]. They also had the lowest alcohol intake although no significance difference was observed across groups.

With the exception of Vitamin D, Groups A, B and C met recommended intakes for all other nutrients including calcium, iron and protein. Dietary intake of Vitamin D intake was low at baseline and 12 weeks for all groups but did not differ over time or across groups (see Table 4). The nutritional parameters and intakes of participants in this study indicated that the population was well nourished and (with the exception of Vitamin D which is gained mainly from sunlight) were consuming adequate nutrients at baseline.

### Blood Data

Concentrations of plasma amino acids at baseline (Table 5 below) were similar with the exception of isoleucine and valine that were significantly higher in group B than in Group C. Moreover isoleucine concentration in group B was significantly higher than in Group A. There was a reduction in the concentration of all 6 measured amino acids in group B following three months of supplementation with the concentrations of isoleucine, valine, alanine and glycine significantly decreased compared to baseline values (see Table 5). There were no significant changes in group A, whilst only glycine was significantly reduced in group C when compared to baseline values (see Table 5). One way ANOVA also showed that there was a significant difference in the mean changes between pre- and post-supplementation in isoleucine and valine. Post-hoc tests showed the mean reductions in both amino acids in group B were significantly different from the mean respective gains in groups A and C (see Table 5). No significant differences were observed between groups A and C.

### DISCUSSION

Despite the fact that the 20% group was receiving as much protein and amino acids as the 40% group through diet and supplementation, changes in LTM over a period of 3 months at 20% leucine (~3g/day) compared to baseline were minimal. At 40% leucine (~6g/day), LTM increased in a sustained manner over the same period. This suggests that an increased dose is required to activate protein synthesis in an elderly age group and that the relative concentration of amino acids in the 20% supplement was not optimised to significantly impact on muscle growth.

The 20% and 40% leucine supplements appear to significantly improve aspects of functional status. It is probable that beneficial adaptations such as changes at the mitochondrial level may occur. Notwithstanding the fact that the subjects were weaker at baseline so that there was greater potential for improvement than in the other two groups, these changes may account for the significant performance gains in the 20% group.

By maintaining the relative proportion of isoleucine and valine present in a standard mixture of EAAs and instructing the subjects to take the supplements alongside food to ensure ample availability of EAAs, the present data suggests that an increased dose of leucine coupled with ample BAAs and EAAs through supplementation and food in an elderly population can be beneficial regarding functional status, maintenance of LTM and ultimately health and quality of life.

Even though there were no significant differences in alcohol consumption between groups, it is noteworthy that % alcohol intake within the EAA supplemented group was three times greater than in the placebo and almost double within the 40% group. Alcohol may have blunted the effects of leucine supplementation on accrual of LTM. The concentration of isoleucine and valine was significantly decreased at the end of the intervention which indicates a decreased rate of protein breakdown as well as an increased rate of protein synthesis.

Surprisingly in the placebo group a small (0.8 kg) but non-significant increase in LTM was not accompanied by significant gains in functional performance. A possible explanation for this discrepancy is that the subjects in the placebo group significantly increased their PA level compared to baseline which stimulated the anabolic role of exercise and increased the utilisation of amino acids for muscle protein synthesis as a result of increased blood flow.

There was also an increase in BMD for both the 20% and 40% groups whilst this was not the case for the placebo group.

### CONCLUSION

The present data supports the potential prophylactic role of leucine and BCAAs for the treatment of sarcopenia. Daily supplementation with approximately 0.21 g/kg/day of EAAs split into two doses and given alongside food can be an effective means for combating sarcopenia together with a diet that provides adequate protein and energy. Supplementation with EAAs resulted in significant gains in physical capacity which is one of the diagnostic criteria for sarcopenia. Moreover supplementation with a mixture of EAAs enriched with 40% leucine and an adequate amount of BCAAs (isoleucine and valine 11% and 12% respectively) resulted in significant gains in functional performance and LTM which was not the case for the standard EAAs and the placebo group. The data suggests that the relative concentration of isoleucine and valine needs to be at least at the same levels as in a standard mixture and be given alongside ample EAAs through food and supplementation for maximum anabolic effects.

| **Table 1:** Age and anthropometric characteristics of the subjects. ^{a} | | | | | | |
|---|---|---|---|---|---|---|
| | | | *Weight* | | *Body Mass Index* | |
| *Group* | *Age (years)* | *Height (m)* | *(kg)* | | *(kg-m⁻²)* | |
| | | | *Baseline* | *12 weeks* | *Baseline* | *12 weeks* |
| *A* | 71.1 ± 2.7 | 1.68 ± 0.11 | 75.6 ± 15.3 | 76.7 ± 15.9 | 26.6 ± 3.8 | 26.8 ± 3.9 |
| *n*=*8 (3M, 5F)* | | | | | | |
| *B* | 71.9 ± 3.0 | 1.67 ± 0.08 | 74.4 ± 12.7 | 74.8 ± 12.2 | 26.7 ± 3.4 | 26.8 ± 3.3 |
| *n*=*8 (4M, 4F)* | | | | | | |
| *C* | 71.8 ± 2.7 | 1.64 ± 0.10 | 70.5 ± 12.4 | 70.8 ± 12.1 | 26.3 ± 3.9 | 26.4 ± 3.9 |
| *n*=*9 (4M, 5F)* | | | | | | |
| *Average* | 71.6 ± 2.7 | 1.66 ± 0.09 | 73.4 ± 13.1 | 74.0 ± 13.1 | 26.5 ± 3.6 | 26.7 ± 3.6 |
| *F (2, 22)* | 0.169 | 0.425 | 0.332 | 0.426 | 0.024 | 0.166 |
| *P-Value* | 0.846 | 0.659 | 0.721 | 0.658 | 0.976 | 0.848 |
| Abbreviations: A= standard essential amino acid mixture (EAAs), B= EAAs containing 40% leucine and, C=placebo. M= male, F=female. ^{a}All values are means ± standard deviation | | | | | | |

| **Table 2:** Assessment of body composition at baseline and end of the intervention period. ^{a} | | | | | | | | | | | |
|---|---|---|---|---|---|---|---|---|---|---|---|
| *Body composition Variables* | *Group A (n=8)* | | | | *Group B (n=8)* | | | | *Group C* (*n*=*9)* | | |
| | *Baseline* | *Week 12* | % | *ES* | *Baseline* | *Week12* | % | *ES* | *Baseline* | *Week 12* | % |
| *Total LTM (kg)* | 44.8±7.1 | 44.8±7.2 | 0.2±2.4 | -0.4 | 45.5±8.9 | 46.0±9.1 | 1.1±1.1* | 0.2 | 42.6±8.9 | 43.0±9.3 | 0.8±1.3 |
| *Total FM (kg)* | 27.8±11.4 | 28.5±11.2 | 4.3±6.7 | 0.4 | 25.6±6.2 | 25.7±6.0 | 0.5±2.6 | -0.2 | 24.9±9.9 | 25.1±9.7 | 1.5±6.3 |
| *Percentage BF* (%) | 36.0±10.5 | 36.7±9.7 | 3.3±6.1 | 0.5 | 34.8±6.6 | 34.7±6.5 | -0.4±2.1 | -0.2 | 35.2±11.2 | 35.2±11.0 | 0.4±4.7 |
| *Total BMC (kg)* | 2.6±0.5 | 2.6±0.5 | 0.1±1.4 | -0.3 | 2.5±0.6 | 2.5±0.6 | 0.0±1.0 | -0.4 | 2.4±0.5 | 2.4±0.5 | 0.4±1.0 |
| *Total BMD (gcm⁻²)* | 1.1±0.1 | 1.1±0.1 | 0.2±1.2 | 0.5 | 1.1±0.2 | 1.1±0.2 | 0.3±1.6 | 0.5 | 1.1±0 .1 | 1.1±0.1 | -0.4± 1.1 |
| Abbreviations: A = standard essential amino acid mixture (EAAs), B= EAAs containing 40% leucine and, C=placebo. M= male, F=female, LTM=bone mineral-free lean tissue mass, FM=fat mass, BMC=bone mineral content, BMD=bone mineral density, BF=body fat, %=mean percentage change from baseline to week 12. All values are means ± standard deviation. * Denotes significantly different from baseline value (* P < 0.05). ME= mean of the experimental group, MP=mean of the placebo group. ES: Effect Size Cohen's d = (*ME-MP)*/*SD pooled*). ^{a} All values are means ± standard deviations | | | | | | | | | | | |

| **Table 3:** Assessment of functional performance and ratings of perceived exertion at baseline and end of the intervention period. ^{a} | | | | | | | | | | | |
|---|---|---|---|---|---|---|---|---|---|---|---|
| *Functional Performance Variables* | *Group A (n=8)* | | | | *Group B (n=8)* | | | | *Group C (n*=*9)* | | |
| | *Baseline* | *Week 12* | % | *ES* | *Baseline* | *Week 12* | % | *ES* | *Baseline* | *Week 12* | % |
| *30-ACT (no)* | 14.6±4.7 | 16.3±4.0 | 15.0±20.0 | 0.8 | 17.4±4.0 | 18.6±3.6 | 8.3±13.7 | 0.6 | 18.6±3.0 | 18.7±3.0 | 0.9±11.2 |
| *30-CST (no)* | 11.6±3.9** | 12.8:1:3.9** | 11.0±11.5* | 0.5 | 14.1±2.0 | 15.9±3.2 | 13.2±16.0 | 0.5 | 16.4±2.1 | 17.1±2.3 | 4.7±15.7 |
| *Relative HST (N kg bw⁻¹)* | 2.8±0.9 | 3.0±1.0 | 11.5±23.9 | 0.6 | 3.8±1.2 | 3.9±1.3 | 4.8±7.5 | 0.6 | 4.0±1.4 | 3.9±1.3 | -0.2:1:8.9 |
| *6-WT (m)* | 501.6±101.1 | 539.0±83.0 | 8.8±10.0* | 0.9 | 533.9±81.0 | 562.1±72.4 | 5.8±6.6* | 0.7 | 586.0±85.0 | 593.0±82.1 | 1.4±4.5 |
| *Average RPE* | 11.3±1.7 | 11.0±1.5 | -2.0±9.0** | 1.3 | 11.7±1.0 | 11.1±1.6 | -5.2±10.1 | 1.0 | 13.1±1.6 | 11.3±1.6 | -13.7±1:5.1* |
| Abbreviations: A= standard essential amino acid mixture (EAAs), B= EAAs containing 40% leucine and, C=placebo. RPE=Rating of Perceived Exertion, %=mean percentage change from baseline to week 12, 30-CST=30-second Chair Stand Test, 30-ACT=30-second Arm Curl Test, 6-WT=6-minute Walk Test, HST= Handgrip Strength Test, no=number of repetitions. * Denotes significantly different from baseline value (* P < 0.05). ** Denotes significantly different from placebo (** P < 0.05) ME= mean of the experimental group, MP=mean of the placebo group. ES: Effect Size Cohen's d = *(ME-MP)*/*SD pooled).* ^{a} All values are means ± standard deviations | | | | | | | | | | | |

| **Table 4:** Nutrient intakes at baseline and at the end of intervention | | | | | | | | | | | | | |
|---|---|---|---|---|---|---|---|---|---|---|---|---|---|
| *Nutritional variables* | *Group A (n=5)* | | | | *Group B (n=8)* | | | | *Group C (n=6)* | | | | *Pre to post suppleme ntation difference across groups (ANOVA)* |
| | *Baseline* | *Week 12* | *Mean Diff* | *p* | *Baseline* | *Week 12* | *Mean Diff* | *p* | *Baseline* | *Week 12* | *Mean Diff* | *p* | *p* |
| *Energy (kcal)* | 1872± 754 | 1811± 740 | -61± 325 | 0.69 | 1787± 305 | 1754 ± 363 | -33.0 ± 175 | 0.67 | 1845 ± 411 | 1812 ± 372 | -33.4 ± 325 | 0.78 | 0.96 |
| *Energy (Kcal) % EAR* | 97.4 ± 35.3 | 94.8 ±33.0 | -2.6 ± 18.1 | 0.77 | 87.5 ± 13.0 | 85.7 ± 12.5 | -1.8± 8.3 | 0.61 | 89.0 ± 24.0 | 88.1± 20.8 | -0.9 ± 17.7 | 0.89 | 0.77 |
| *Total* % *fat* | 34.6 ± 12.3 | 28.2 ± 8.6 | -6.4 ± 7.3 | 0.12 | 32.7 ± 5.0 | 31.5 ± 2.3 | -1.2 ± 5.8 | 0.64 | 31.5 ± 5.2 | 32.3 ± 5.3 | 0.8± 6.5 | 0.75 | 0.98 |
| *Total* % *CHO* | 36.9 ± 6.9 | 43.1± 3.1 | 6.2 ± 6.8 | 0.11 | 41.7 ± 3.0 | 42.2 ± 8.2 | 0.5 ± 2.7 | 0.85 | 46.9 ± 6.0 | 47.8 ± 5.4 | 0.9 ± 3.3 | 0.44 | 0.05 |
| *Total* % *protein* | 14.1± 2.7 | 16.5 ± 4.7 | 2.4 ± 4.5 | 0.29 | 17.4 ± 3.8 | 18.2 ± 3.9 | 0.8 ± 5.5 | 0.76 | 16.9 ± 2.4 | 16.1 ± 3.1 | -0.8.± 2.6 | 0.45 | 0.34 |
| *Total* % *alcohol* | 14.3 ± 17.4 | 12.1± 13.6 | -1.8 ± 4.1 | 0.29 | 8.2 ± 6.1 | 8.2 ± 4.5 | 0 ± 4.7 | 0.97 | 4.7 ± 3.6 | 3.8 ± 4.3 | -0.9 ± 2.9 | 0.38 | 0.21 |
| *Protein (g)* | 62.5 ± 17.4 | 71.2 ± 30.2 | 8.7 ± 15.0 | 0.27 | 77.5 ± 18.4 | 72.3 ± 15.9 | -5.2 ± 16.7 | 0.40 | 77.8 ± 19.2 | 78.6 ± 20.2 | 0.8 ± 30.7 | 0.95 | 0.54 |
| *Protein(% RNI)* | 133.2 ± 37.9 | 152.2 ± 65.3 | 19.0 ± 32.2 | 0.26 | 166 ± 40.4 | 167 ± 44.1 | 1.8± 65.7 | 0.95 | 165.6 ± 39.1 | 150.1± 40.8 | -15.6± 39.8 | 0.31 | 0.57 |
| *Calcium (% RNI)* | 121± 65.4 | 108 ± 65.3 | -13.0 ± 28.6 | 0.37 | 109 ± 42.4 | 107.7 ± 317 | -1.3 ± 44.3 | 0.94 | 130 ± 42.0 | 121± 41.1 | -9. ± 23.0 | 0.30 | 0.80 |
| *Iron (% RNI)* | 123.6 ± 65.0 | 123.2 ± 103.7 | 0.4 ± 73.2 | 0.99 | 98.6 ± 1.2 | 96.3 ± 35.0 | -2.3.± 43.0 | 0.66 | 116 ± 34.5 | 110 ± 43.7 | -6.0 ± 28.6 | 0.56 | 0.97 |
| *Vitamin D (% RNI)* | 20.6 ± 19.4 | 39.8 ± 64.0 | 19.2 ± 68.2 | 0.56 | 13.7 ± 5.5 | 46 ± 51.2 | 32.3 ± 50.8 | 0.20 | 34.5 ± 14.0 | 29.5 ± 19.5 | -5.± 29.7 | 0.54 | 0.34 |
| Abbreviations: A= standard essential amino acid mixture (EAAs), B= EAAs containing 40% leucine and, C=placebo, EAR=Estimated average requirement, RNI=Reference Nutrient Intake. All values are means ± standard deviation. | | | | | | | | | | | | | |

| **Table 5:** Concentration of amino acids (µMol·L⁻¹) at baseline and at the end of intervention | | | | | | | | | | |
|---|---|---|---|---|---|---|---|---|---|---|
| *Amino acids* | *Group A (n=3)* | | | *Group B (n=4)* | | | *Group C (n=6)* | | | *Pre to post supplementation difference across groups (ANOVA)* |
| | *Baseline* | *Week 12* | *Mean Diff* | *Baseline* | *Week 12* | *Mean Diff* | *Baseline* | *Week 12* | *Mean Diff* | *p* |
| *Leucine* | 147.3±8.1 | 175.3±24.6 | 28.0±29.1 | 224.0 ± 82.5 | 187.0±21.5 | - 37.0±75.3 | 142.5 ± 36.4 | 145.8±23.0 | 3.3±33.3 | 0.250 |
| *Isoleucine* | 64.7±7.6 | 78.7±6.0 | 14.0±14.0 | 101.7 ± 24.0**§ | 65.3±9.6* | - 36.5±15.4 | 61.7 ± 10.8 | 66.2±11.4 | 4.5±14.7 | **0.002** |
| *Valine* | 192.0±23.6 | 219.7±43.7 | 27.7±46.1 | 259.5 ± 41.5** | 203.8±39.7* | - 55.8±27.6 | 175.8 ± 42.1 | 198.5±37.0 | 22.7±39.3 | **0.017** |
| *Alanine* | 223.0±93.2 | 250.7±84.8 | 27.7±84.2 | 254.5 ± 47.7 | 212.3±54.4* | - 42.3±23.0 | 241.3 ± 46.1 | 259.7±39.1 | 18.3±33.8 | 0.119 |
| *Glycine* | 180.0±51.6 | 172.7±110.0 | -7.3±59.1 | 186.0 ± 31.4 | 156.3±17.9* | - 29.8±18.5 | 193.2 ± 73.1 | 223.5±96.7* | 30.3±28.1 | 0.059 |
| *Proline* | 141.0±33.9 | 140.3±5.8 | -0.7±39.0 | 132.5 ± 12.7 | 114.8±22.9 | - 17.8±26.0 | 112.5 ± 26.8 | 128.0±40.0 | 15.5±32.2 | 0.310 |
| Abbreviations: A= standard essential amino acid mixture (EAAs), B= EAAs containing 40% leucine and, C=placebo. All values are means ± standard deviation. * Denotes significantly different from baseline value (P < 0.05). ** Denotes significantly different from placebo (P < 0.05). § Denotes significantly different from group A (P < 0.05) | | | | | | | | | | |

Viewed from a first aspect the present invention provides a dietary supplement comprising a dry blend of essential amino acids being:
leucine in an amount in excess of 20wt% of the dry blend;
isoleucine and valine in a combined amount of 23wt% or more of the dry blend; and additional essential amino acids.

Preferably leucine is present in an amount of 40wt% or more of the dry blend.

Preferably the ratio of the amount of leucine to the combined amount of isoleucine and valine is greater than 0.870.

Preferably the ratio of the amount of leucine to the combined amount of isoleucine and valine is 1.739 or more.

Preferably the amount of isoleucine is 1 1wt% or more of the dry blend.

Preferably the amount of valine is 12wt% or more of the dry blend.

Preferably the additional essential amino acids are histidine, lysine, methionine, threonine and phenylalanine.

Viewed from a further aspect the present invention provides dietary supplement as defined hereinbefore for use by oral administration to a subject in combatting degenerative loss of a muscle characteristic in the subject.

The dietary supplement for use is preferably by oral administration to the subject of doses of the dry blend in the range 0.18 to 0.24 g/kg (body weight) per day.

The dietary supplement for use is preferably by oral administration to the subject twice daily.

The dietary supplement for use is preferably by oral administration to the subject with food.

In the dietary supplement for use, the subject is preferably receiving dietary protein in the range 1.0 to 1.3 g/kg (body weight) per day.

The dietary supplement for use is preferably by oral administration to the subject over a period of 12 weeks or more.

Viewed from a yet further aspect the present invention provides a method of treatment of a subject to combat degenerative loss of a muscle characteristic in the subject comprising: orally administering to the subject a dietary supplement as hereinbefore defined.

## Claims

1. Dietary supplement for use by oral administration to a subject in combatting degenerative loss of a muscle characteristic in the subject, wherein the dietary supplement comprises a dry blend of essential amino acids being:
leucine in an amount in excess of 20wt% of the dry blend;
isoleucine and valine in a combined amount of 23wt% or more of the dry blend; and
additional essential amino acids.

2. Dietary supplement for use as claimed in claim 1 in combatting sarcopenia in the subject, wherein oral administration is twice daily and is of doses of the dry blend in the range 0.18 to 0.24 g/kg (body weight) per day, wherein the dietary supplement consists essentially of a dry blend of essential amino acids being:
leucine in an amount of 40wt% or more of the dry blend;
isoleucine and valine in a combined amount of 23wt% or more of the dry blend; and
additional essential amino acids
wherein the dietary supplement is orally administered to the subject with food.

3. Dietary supplement for use as claimed in claim 1 or 2, wherein the subject is receiving dietary protein in the range 1.0 to 1.3 g/kg (body weight) per day.

4. Dietary supplement for use as claimed in any preceding claim by oral administration to the subject over a period of 12 weeks or more.

5. Dietary supplement for use as claimed in any preceding claim wherein the ratio of the amount of leucine to the combined amount of isoleucine and valine is greater than 0.870.

6. Dietary supplement for use as claimed in any preceding claim wherein the ratio of the amount of leucine to the combined amount of isoleucine and valine is 1.739 or more.

7. Dietary supplement for use as claimed in any preceding claim wherein the amount of isoleucine is 1 1wt% or more of the dry blend.

8. Dietary supplement for use as claimed in any preceding claim wherein the amount of valine is 12wt% or more of the dry blend.

9. Dietary supplement for use as claimed in any preceding claim wherein the additional essential amino acids are histidine, lysine, methionine, threonine and phenylalanine.
